# EUROPEAN PATENT APPLICATION

(11) **EP 4 331 596 A1**
(43) Date of publication of application: **06.03.2024**
(21) Application number: 22382821.1
(22) Date of filing: 02.09.2022
(51) Int. Cl.: A61K 35/74, A61K 45/06, A61P 17/10, A61P 31/04, A61Q 17/00, A61Q 19/00

(54) **FUSOBACTERIUM NUCLEATUM FOR TREATING ACNE**

(71) Applicant: Universitat Internacional De Catalunya, 08017 Barcelona (ES)
(72) Inventor: CLOTET ERRA, Josep, 08222 TERRASSA (ES); JIMÉNEZ JIMÉNEZ, Javier, 08198 ST. CUGAT VALLÈS (ES); GRIMALT SANTACANA, Ramon, 08221 TERRASSA (ES); MARTÍNEZ LÁINEZ, Joan Marc, 08203 SABADELL (ES)
(74) Representative: ZBM Patents - Zea, Barlocci & Markvardsen

(57) **Abstract**

The present invention provides *Fusobacterium nucleatum,* or a lysate thereof, or a culture supernatant thereof, for use in the prevention and/or treatment of a bacterial infection. The invention also provides a pharmaceutical composition and a cosmetic composition comprising *Fusobacterium nucleatum,* or a lysate thereof, or a supernatant thereof; and a non-therapeutic use of *Fusobacterium nucleatum,* or a lysate thereof, or a supernatant thereof, as an antibacterial agent.

## Description

### Technical Field

The present invention belongs to the field of medicine, in particular, treatments of bacterial infections. The compositions of the invention are particularly useful for treating infections related to *Cutibacterium acnes,* in particular acne vulgaris.

### Background Art

*Cutibacterium acnes* -formerly named *Propionibacterium acnes-* is a Gram-positive bacterium that forms part of the normal microbiota of the skin, oral cavity, large intestine, conjunctiva, and external ear canal.

*C. acnes* is also associated with pathologic conditions, such as acne vulgaris (acne), and it is an important opportunistic pathogen. In this regard, *C. acnes* has been described to play a role in a wide variety of infections, such as dental infections, endocarditis, joint infection, central nervous system infections, ocular infections and sepsis. Notably, *C. acnes* is known to cause several postoperative and device-related infections, such as infections related to joint prostheses, and prosthetic heart valves.

Acne is one of the most prevalent skin diseases and it usually affects young adults. Nearly 80% of teenagers have presented with acne. Acne is characterized by *C. acnes* hyperproliferation, an increased secretion of sebum, and an aberrant growth of the epidermis and hair follicles. Acne causes important problems as the resulting skin appearance can lead to anxiety, reduced self-esteem, and, in extreme cases, depression.

Infections caused by *C. acnes,* including acne, are usually treated with antibiotics that, although generally safe, often generate side effects including nausea, fatigue, dizziness, vomiting, and photosensitivity. Also importantly, the worldwide prevalence of antibioticresistant *C. acnes* is increasing, which is leading to a reduction in the efficacy of current anti-*C. acnes* treatments.

Therefore, in spite of the efforts made so far, there is still a need for novel treatments of bacterial infections, particular those associated with *C. acnes,* which are effective and do not cause undesired secondary effects.

### Summary of Invention

The present inventors have surprisingly found that *Fusobacterium nucleatum* produces extracellular metabolites with a remarkable antibacterial activity.

As shown in the examples below, *F. nucleatum* cells or their culture supernatant have a remarkable capacity to inhibit bacterial growth, in particular *Cutibacterium acnes* growth, which is an important opportunistic bacterium that causes a wide-range of infections. Importantly, *C. acnes* is one of the causative agents of acne vulgaris, one of the most common skin diseases.

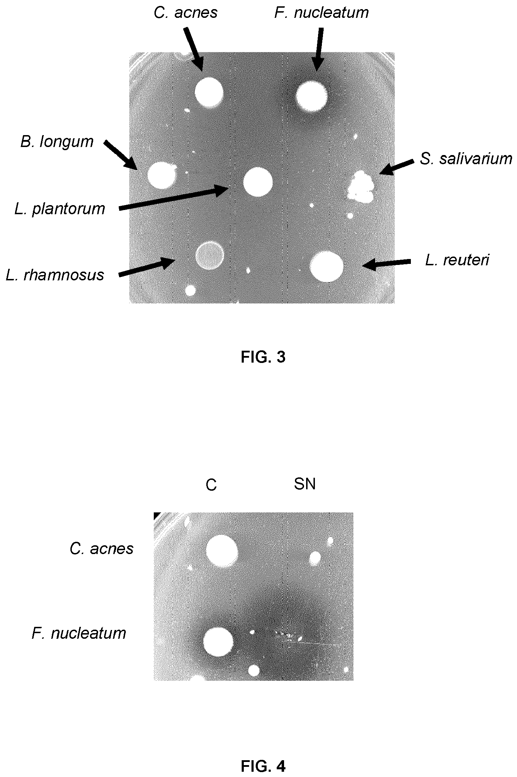

The examples below show that the antimicrobial effect of *F. nucleatum* is stronger than the effect of known probiotic bacterial species such as *Bifidobacterium longum, Lactobacillus plantarum, Streptococcus salivarius, Lactobacillus rhamnosus* (see Figure 3). Also, the inhibitory activity of *F. nucleatum* is maintained after freeze-drying the supernatant, which makes it highly suitable for long-term storage and facilitates its handling and use (Figure 4).

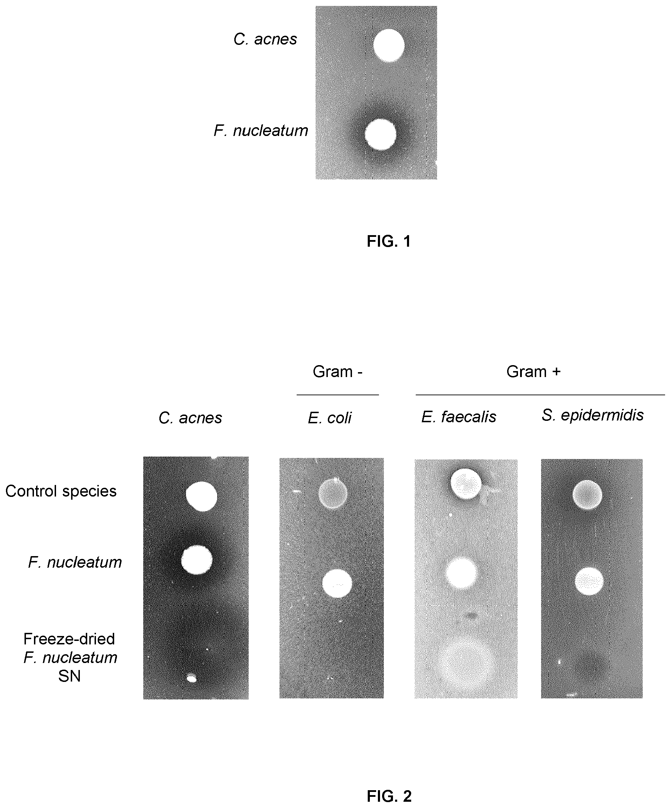

Furthermore, the present inventors also found that the bacterial inhibitory capacity of *F*. *nucleatum* is highly specific against *C*. *acnes,* as it does not affect the growth of other bacterial species, in particular those forming the normal microbiota of the skin -such as *Staphylococcus epidermidis* (see Figure 2). Thus, contrary to the known antibacterial agents that also alter the beneficial microbiota of the body, the present invention provides a treatment of bacterial infections which is efficient and has reduced secondary effects. This is particularly important in the case of anti-acne treatments, most of which currently entail important undesired effects on the skin.

Advantageously, the antibacterial effect of *F. nucleatum* can also be achieved by its cell-free supernatant, thereby reducing any risk associated to the use of live bacterial cells.

Altogether, the present invention provides a great advance in the field of antibacterial agents and the treatment of bacterial infections.

Thus, in a first aspect, the invention provides *Fusobacterium nucleatum,* or a lysate thereof, or a culture supernatant thereof, for use in the prevention and/or treatment of a bacterial infection.

This aspect can also be formulated as the use of *Fusobacterium nucleatum,* or a lysate thereof, or a culture supernatant thereof for the manufacture of a medicament for the treatment or prevention of a bacterial infection. This aspect can also be formulated as a method for treating or preventing a bacterial infection, the method comprising administering a therapeutically effective amount of *Fusobacterium nucleatum,* or a lysate thereof, or a culture supernatant thereof together with pharmaceutically acceptable excipients or carriers to a subject in need thereof.

In a second aspect, the invention provides a pharmaceutical composition comprising a therapeutically effective amount of the *Fusobacterium nucleatum,* or a lysate thereof, or a supernatant thereof together with at least one pharmaceutically acceptable excipient and/or carrier.

In a third aspect, the invention provides the pharmaceutical composition as defined in the second aspect, for use as a medicament.

In a fourth aspect, the invention provides the pharmaceutical composition as defined in the second aspect, for use in the prevention and/or treatment of a bacterial infection.

The non-pathogenic growth of *Cutibacterium* species has also been related to cosmetic issues, such as body odor.

Thus, in a fifth aspect, the invention provides a cosmetic composition comprising a cosmetically effective amount of *Fusobacterium nucleatum,* or a lysate thereof, or a supernatant thereof, together with at least one cosmetically acceptable excipient and/or carrier.

In a sixth aspect, the invention provides a non-therapeutic use of *Fusobacterium nucleatum,* or a lysate thereof, or a supernatant thereof, in cosmetics.

Inhibition of bacterial growth by *F. nucleatum* also opens a window in industrial fields besides therapeutics. It is known that *C*. *acnes* can form biofilms, and that bacterial biofilms may be formed in many surfaces, in particular abiotic surfaces. Also, C. acnes has been found to live as an endophyte in plants.

Thus, in a seventh aspect, the invention provides a non-therapeutic use *Fusobacterium nucleatum,* or a lysate thereof, or a supernatant thereof, as an antibacterial agent.

### Brief Description of Drawings

Fig. 1 shows a picture of the inhibition zones generated by the indicated bacterium (C. *acnes* or *F. nucleatum*) on a *C*. *acnes* culture. *C*. *acnes* was embedded in a BMSA plate as described in the examples below. 10 µl of the indicated *F. nucleatum* at a 50 OD/ml concentration were spotted on the plate and incubated for 2 to 3 days at 37°C in anaerobic conditions. The dark zone around *F. nucleatum* corresponds with a *C*. *acnes* growth inhibition zone.
Fig. 2 shows pictures of the inhibition zones generated on the indicated cultures by *F. nucleatum* cells, reconstituted freeze-dried *F. nucleatum* supernatant (SN), or a control bacterium (which is *C. acnes* on the C. *acnes* culture, *E. coli* on the *E. coli* culture, *E. faecalis* on the *E. faecalis* culture, and *S. epidermis* on the *S. epidermis* culture). Dark zones around the spotted *F. nucleatum* cells or SN correspond with a *C. acnes* growth inhibition zone.
Fig. 3 shows a picture of the inhibition zones generated by the indicated probiotic bacterium on a *C. acnes* culture. Probiotic species were grown in MRS and 10 µl were spotted on a DMSA plate containing *C. acnes.* The plate was incubated for 2 to 3 days at 37°C in anaerobic conditions. Abbreviations are for *Bifidobacterium longum, Lactobacillus plantarum, Streptococcus salivarius, Lactobacillus rhamnosus,* and *Lactobacillus reuteri.* Dark zones around the spotted *F. nucleatum* correspond with a *C. acnes* growth inhibition zone.
Fig. 4 shows a picture the inhibition zones generated by the indicated bacterial cells (C) or by reconstituted freeze-dried supernatant (SN) on a *C. acnes* culture. *F. nucleatum* and *C. acnes* (as control species) were grown in liquid BM. The culture (50 ml) was centrifuged and filtered to eliminate all cells and freeze-dried was solved in 1 ml of water. 10 µl was spotted on a DMSA plate containing *C. acnes.* The plate was incubated for 2 to 3 days at 37°C in anaerobic conditions. Dark zones around the spotted *F. nucleatum* cells or reconstituted freeze-dried supernatant correspond with a *C. acnes* growth inhibition zone.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

*Fusobacterium nucleatum* (*F. nucleatum*) is a Gram negative, non-spore forming anaerobic bacterium. It is a prominent member of the commensal flora of the human oral cavity, along with a number of other anaerobes. *F. nucleatum* is commonly associated with the formation of dental plaque biofilms. Several commercially available and deposited strains of *F. nucleatum* are available and can be used in the present invention, for example, *Fusobacterium nucleatum subsp. polymorphum* strain ATCC10953.

As used herein, "culture supernatant" or "conditioned culture media" refer to culture media in which *Fusobacterium nucleatum* cells have been cultured for a suitable period of time and into which the cells have secreted active agent(s) to sufficient levels to provide a desired biological activity, in particular, *C. acnes* growth inhibition. A culture supernatant may contain one or more soluble metabolites of the bacterium useful in the methods described herein. The skilled in the art will know which media is suitable for culturing the bacterium, and for how long it should be cultured in order to obtain a supernatant with enough active metabolites using its common general knowledge. The culture supernatant may be obtained, for example, by settling, precipitating or centrifuging the cells in culture. The supernatant may be filtered. A culture supernatant may contain one or more soluble metabolites.

As used herein "lysate" refers to a sample of *F. nucleatum* which has been subject to lysis. A lysate may contain one or more soluble metabolites of the bacterium useful in the methods described herein. The skilled person knows several methods to carry out the lysis which belong to the common general knowledge. For example, lysis may occur by chemical or physical disruption, such as by addition of an osmotic agent or enzyme to the bacteria, or by the application of physical pressure, for example through sonic disruption. The bacteria may be washed prior to lysis. The lysate may be filtered and/or sterilized after lysis to remove any whole bacteria remaining. The lysate may be suspended, for example in aqueous medium.

The term "bacterial infection" refers to a condition or disease associated with the proliferation of a pathogenic bacteria on or inside the body. Pathogenic bacteria can be opportunistic bacteria, that is bacteria that are normally commensal in the body -such as *C*. *acnes-* but that can cause disease under particular circumstances -such as acne when hair follicles get clogged.

As used herein, "acne vulgaris" or "acne" refers to a skin condition that occurs when dead skin cells and oil from the skin clog hair follicles. Acne may be classified as non-inflammatory acne -when there is no bacterial infection- and inflammatory acne -when the clogged pores have become infected with bacteria, in particular *C. acnes.*

The term "pharmaceutically acceptable" as used herein pertains to compounds, materials, compositions, and/or dosage forms which are, within the scope of sound medical judgment, suitable for use in contact with the tissues of a subject (e.g., human) without excessive toxicity, irritation, allergic response, or other problem or complication, commensurate with a reasonable benefit/risk ratio. Each carrier, excipient, etc., must also be "acceptable" in the sense of being compatible with the other ingredients of the formulation. Suitable carriers, excipients, etc. can be found in standard pharmaceutical texts, and include, as a way of example preservatives, agglutinants, humectants, emollients, and antioxidants.

The term "effective amount" as used herein, means an amount of an active agent high enough to deliver the desired benefit (either the treatment or prevention of the illness), but low enough to avoid serious side effects within the scope of medical judgment.

The terms "cosmetically acceptable" or "dermatological acceptable" have the same meaning and which are herein used interchangeably. They refer to that excipients or carriers suitable for use in contact with human skin without undue toxicity, incompatibility, instability, allergic response, among others. The excipients or carriers used have affinity for the skin, are well tolerated, stable, and are used in an amount adequate to provide the desired consistency, and ease application. The appropriate excipients and/or carriers, and their amounts, can readily be determined by those skilled in the art according to the type of formulation being prepared to provide the desired consistency and ease application. Examples of appropriate excipients or carriers can include, but not limited to, surfactant, vehicle, preservative, propellant, pH-regulating agent, stabilizing agents, viscosity agent, skin conditioning agent, antioxidant and mixture thereof. The bacterium, lysate or supernatant of the invention can also be cosmetic in case that the skin is not considered to suffer any disease, but due to the presence, for example, of comedo or whiteheads (without inflammation) an unaesthetic appearance is made evident.

The term "abiotic surface" is to be understood as any surface which is different from a surface of a living body (i.e., skin, mucosa).

As indicated above, the invention provides in a first aspect *Fusobacterium nucleatum,* or a lysate thereof, or a culture supernatant thereof, for use in the prevention and/or treatment of a bacterial infection. In other words, the invention provides *Fusobacterium nucleatum* cells, or *Fusobacterium nucleatum* lysate, or *Fusobacterium nucleatum* culture supernatant, for use in the prevention and/or treatment of a bacterial infection. *Fusobacterium nucleatum* cells can be provided in the form of a cell culture or in purified form.

In a particular embodiment of the first aspect, the bacterial infection is caused by or associated with *Cutibacterium acnes.* In other words, the bacterial infection is related to the pathogenic growth of *Cutibacterium acnes.* In a more particular embodiment, the bacterial infection caused by or associated with *Cutibacterium acnes* is selected from the group consisting of acne vulgaris, ocular infection (e.g. blepharitis and endophthalmitis), dental infection, skin infection (e.g. fatal bacteria granuloma after trauma), endocarditis, bone infection, joint infection, central nervous system infection (e.g. spondylodiscitis or cerebrospinal fluid shunt infection), sepsis, postoperative infection, cerebrospinal fluid shunt infection, device-related infection, and combinations thereof.

In a particular embodiment of the first aspect, *Fusobacterium nucleatum,* or a lysate thereof, or a culture supernatant thereof, is for the prevention and/or treatment of acne vulgaris. In a more particular embodiment, acne vulgaris is inflammatory acne.

In a particular embodiment of the first aspect, *Fusobacterium nucleatum* is in the form of an isolated bacterial population; particularly an isolated bacterial strain.

In a particular embodiment of the first aspect, *Fusobacterium nucleatum* is alive, attenuated, inactivated, or dead.

In a particular embodiment of the first aspect, *Fusobacterium nucleatum* culture supernatant is a cell-free supernatant and/or the *Fusobacterium nucleatum* lysate is a cell-free lysate.

In a particular embodiment of the first aspect, the lysate is obtainable by a method comprising, consisting essentially of, or consisting of, the steps a) culturing the *Fusobacterium nucleatum* under suitable growth conditions, particularly anaerobic conditions, thereby generating a cell culture; and b) lysing the cell culture thereby obtaining the lysate. For the purposes of the invention, the expressions "obtainable", "obtained" and equivalent expressions are interchangeably used, and in any case the expression "obtainable" includes the expression "obtained".

In a particular embodiment of the first aspect, the lysate is obtainable by a method comprising, consisting essentially of, or consisting of, the steps a) culturing the *Fusobacterium nucleatum* under suitable growth conditions, particularly anaerobic conditions, thereby generating a cell culture; b) lysing the cell culture thereby obtaining the lysate, and c) optionally, further subjecting the lysate of step (b) to a step selected from the group consisting of dehydration, filtration, ultra-filtration, centrifugation, ultra-centrifugation, precipitation, chromatography, and combinations thereof.

In a particular embodiment of the first aspect, the *Fusobacterium nucleatum* supernatant is obtainable by a method comprising, consisting essentially of, or consisting of, the steps a) culturing the *Fusobacterium nucleatum* in a culture medium under suitable growth conditions, particularly anaerobic conditions; and b) separating the culture medium from the *Fusobacterium nucleatum* cells, thereby obtaining the culture supernatant. In a more particular embodiment, the *Fusobacterium nucleatum* is cultured for a suitable period of time, particularly for at least 12h, 24h, 48h, 60h, or 72h. In an even more particular embodiment, the *Fusobacterium nucleatum* is cultured for about 48h. In a particular embodiment, the separation of the culture medium from the cells is carried out by centrifugation and/or filtration. In another particular embodiment, the culture supernatant is further subjected to a step selected from the group consisting of dehydration, filtration, ultra-filtration, centrifugation, ultra-centrifugation, precipitation, chromatography, and combinations thereof.

In a particular embodiment of the first aspect, *Fusobacterium nucleatum* lysate or supernatant is sterilized, particularly filter sterilized.

In a particular embodiment of the first aspect, the *Fusobacterium nucleatum* strain is a non-pathogenic strain of *Fusobacterium nucleatum.* In a more particular embodiment, the *Fusobacterium nucleatum* strain is a probiotic strain.

In a particular embodiment of the first aspect, the *Fusobacterium nucleatum,* a lysate thereof, or a supernatant thereof, is concentrated and/or freeze-dried. In a more particular embodiment, is reconstituted freeze-dried.

In a particular embodiment of the first aspect, the *Fusobacterium nucleatum* is selected from the group of subspecies consisting of *Fusobacterium nucleatum subsp. polymorphum, Fusobacterium nucleatum subsp. nucleatum, Fusobacterium nucleatum subsp. vincentii, Fusobacterium nucleatum subsp. fusiforme,* and *Fusobacterium nucleatum subsp. animalis.* In a more particular embodiment, *Fusobacterium nucleatum* is not *Fusobacterium nucleatum subsp. nucleatum.* In an even more particular embodiment, the *Fusobacterium nucleatum* is *Fusobacterium nucleatum subsp. polymorphum.*

In a particular embodiment of the first aspect, the *Fusobacterium nucleatum subsp. polymorphism* is the strain ATCC10953 or a mutant thereof which retains or enhances the capacity of the parent strain to inhibit *C*. *acnes* growth. In another particular embodiment, the *Fusobacterium nucleatum subsp. polymorphum* is the strain ATCC10953 or a mutant strain obtained from the ATCC10953 strain which retains or enhances the capacity of the parent strain to inhibit *C. acnes* growth. In a more particular embodiment, the strain ATCC10953 is a strain with the genome sequence with the NCBI accession number NZ_AARG00000000, version NZ_AARG00000000.1.

An expert in the art will understand that using any strain as starting material it is routinely possible to obtain, for example by spontaneous mutation or directed mutagenesis, mutants that retain the characteristics and relevant advantages described herein. Methods for obtaining mutants of a given bacterial strain are known in the art. The skilled in the art would also know how to test the capacity of a mutant strain to inhibit *C. acnes* growth using common general knowledge, for example, by carrying out an inhibition zone assay as described in the examples below -i.e., pipetting the mutant strain on a *C. acnes* culture and measuring the inhibition zone generated.

In a particular embodiment of the first aspect, the *Fusobacterium nucleatum,* or a lysate thereof, or a culture supernatant thereof, is for use in combination therapy with an antibiotic or an anti-inflammatory agent. Antibiotics and anti-inflammatory agents that can be used in the present invention, in particular for treating *C. acnes* infections, are well known to the skilled person and form part of the common general knowledge.

In a further aspect, the invention provides the *Fusobacterium nucleatum,* or a lysate thereof, or a supernatant thereof, for use in the treatment of a disease or condition caused by or associated with *Cutibacterium acnes,* particularly acne vulgaris.

In a further aspect, the invention provides the *Fusobacterium nucleatum,* or a lysate thereof, or a supernatant thereof, for use in the treatment of a disease or condition caused by or associated with *Cutibacterium acnes,* particularly a *Cutibacterium acnes* infection.

As above described, the invention also provides in a second aspect a pharmaceutical composition comprising a therapeutically effective amount of the *Fusobacterium nucleatum,* or a lysate thereof, or a supernatant thereof together with at least one pharmaceutically acceptable excipient and/or carrier. All embodiments described above for the first aspect regarding the *Fusobacterium nucleatum,* or a lysate thereof, or a supernatant thereof, are also meant to apply to this second aspect.

In a particular embodiment of the second aspect, the pharmaceutical composition further comprises a bacterium of a species different from *Fusobacterium nucleatum,* or a lysate thereof, or a supernatant thereof. In a more particular embodiment, the pharmaceutical composition comprises a probiotic bacterium different from *F. nucleatum.*

As above described, in a third aspect the invention provides the pharmaceutical composition of the second aspect for use as a medicament. In a fourth aspect, the invention provides the pharmaceutical composition of the second aspect for use in the prevention and/or treatment of a bacterial infection. All the embodiments of the first aspect regarding the prevention and/or treatment of a bacterial infection are also meant to apply to the fourth aspect of the invention.

This aspect can also be formulated as the use of the pharmaceutical composition of the invention for the manufacture of a medicament for the treatment or prevention of a bacterial infection. This aspect can also be formulated as a method for treating or preventing a bacterial infection, the method comprising administering a therapeutically effective amount of the pharmaceutical composition of the invention to a subject in need thereof.

In a particular embodiment of the fourth aspect, the prevention and/or treatment comprises administering the pharmaceutical composition at a dose causing the inhibition or reduction of *C. acnes* growth.

As above indicated, in a fifth aspect, the invention provides a cosmetic composition comprising a cosmetically effective amount of *Fusobacterium nucleatum,* or a lysate thereof, or a supernatant thereof, together with at least one cosmetically acceptable excipient and/or carrier. In a particular embodiment, the cosmetic composition is a deodorant cosmetic composition.

In a particular embodiment of the second and fifth aspects, the pharmaceutical or cosmetical composition is formulated for topical use, particularly in a form selected from the group consisting of cream, gel, oil, emulsion, spray, gauze, patch, bandage, lotion, mousse, ointment, paste, liquid formulation for extemporaneous preparations, and mixtures thereof.

In a particular embodiment of the second or fifth aspects, the pharmaceutical or cosmetical composition is formulated for oral administration, particularly as a solid formulation, optionally in the form of a pill, capsule, table, granular powder.

As indicated before, the invention also provides in a sixth aspect a non-therapeutic use of *Fusobacterium nucleatum,* or a lysate thereof, or a supernatant thereof, in cosmetics.

In a particular embodiment of the sixth aspect, the non-therapeutic use is for treating non-inflammatory acne. In another particular embodiment of the sixth aspect, the non-therapeutic use is for reducing body odor.

In a seventh aspect, the invention provides a non-therapeutic use of *Fusobacterium nucleatum,* or a lysate thereof, or a supernatant thereof, as an antibacterial agent.

In a particular embodiment of the seventh aspect, the use of *Fusobacterium nucleatum,* or a lysate thereof, or a supernatant thereof, is for inhibiting *C. acnes* growth.

In a particular embodiment of the seventh aspect, the use of *Fusobacterium nucleatum,* or a lysate thereof, or a supernatant thereof is as antibacterial agent in an abiotic surface or in a plant.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Examples

### Materials and methods

### Acne patients sampling

40 individuals aged 14-15 from the same school in the area of EI Vallès around Barcelona (Spain) were informed about this study. Informed consent was asked to their parents or legal tutors. Exclusion criteria were applied (smokers, antibiotic treatment during the last 6 months, benzoyl peroxide treatment during the last 6 months, dermatological disease affecting the sampling area). A dermatologist assessed the acne severity, including grade 1 and 2 individuals only. Samples were collected by swapping 4 times a constant area on the chin, both cheeks and forehead using a sterile swab (Catch-Ail^{™} ref. QEC091H from Epicentre) previously soaked in 0.15M NaOH solution containing 0.01% Tween 20 as described in Fierer *et al.,* 2010. Briefly, the swab was included and violently stirred into an Eppendorf tube containing 0.5 ml of PBS. Samples were kept on ice and immediately frozen at -20°C upon arrival in the lab. DNA extraction was done using the Norgen saliva DNA isolation kit (ref: RU454000), following manufacturer's instructions.

### Culture media

BHI (Brain Heart Infusion broth) was from Conda (ref 1048). R-2A (Reasoner's 2A) was from Condalab (ref: 1071.00). Nissui is a generally used nickname for GAM Broth Modified produced by Nissui (ref 05433). BM (Bob Medium): yeast extract 2.5 g/l (Condalab ref: 1702.00), meat peptone 2.5 g/l (Condalab ref: 1616.05), casein hydrolysate 2.5 g/l (Merck ref: 22090), glucose 2.5 g/l, dipotassium hydrogen phosphate 1.5 g/l (Panreac ref 121512.1211) and sodium pyruvate 1.5 g/l (Gibco ref: 11840-048)). HEPES (VWR ref: 0511) was added to a final concentration of 75 mM. pH was adjusted to 7 by adding NaOH (Panreac ref: 141687.1211). MRS medium was from Scharlau (ref:01-135-500).

BMSA was BM containing 0.7% agar-agar (BM Soft Agar). BMSA was autoclaved and kept at 45°C, to include *C. acnes* at a final concentration of 0.0025 OD₆₀₀/ml (1OD is the number of bacteria that when in 1 ml produces 1 unit of absorbance). The medium was laid in Petri dishes for solidification and ulterior use.

### Inhibition zone assay

All different bacteria strains were thawed from the glycerol stock stored at -80°C. Particularly, *F. nucleatum* was streaked on agar-Nissui plates and *C. acnes* on agar-BHI plates. Bacteria were grown for 2 to 3 days at 37°C into an anaerobic jar (AnaeroGen, OXOID 2004AN0025A). *C. acnes* cells were scraped and placed in 1 ml of BM medium. Optical density was measured using a spectrophotometer at a wavelength of λ=600 nm. To embed *C. acnes* in the BMSA plates, *C. acnes* at a final concentration of 0.0025 OD_{λ600}/ml was added to the medium kept in a water bath at 45°C and disposed of in empty 10 cm Petri plates and let them solidify into a hood for 15 min. The Petri plate lids were partially open to avoid excess water condensation.

In parallel, *F. nucleatum* plate was scraped and the cells were resuspended in BM medium, cells were harvested by centrifugation (14,100 rcf for 1 min) and resuspended in BM medium to a final concentration of 50 OD _{λ600}/ml.

To check the inhibition capability of *F. nucleatum* cells by the formation of inhibition zones, 10 µl of *F. nucleatum* in the above conditions were pipetted over the embedded *C. acnes* plate described above. The plate was left for 15-30 min in the hood for the liquid to be absorbed. Plates were kept at 37°C in anaerobic conditions for 2 to 3 days.

To check the inhibition zone produced by the medium where *F. nucleatum* has been grown (i.e., the culture supernatant), 0.06 OD_{λ600} of *F. nucleatum* were inoculated on 150 ml of liquid BM medium in a 250 ml flask. The flask was kept in anaerobic condition and without agitation for 2 to 3 days at 37°C. The culture was split into 50 ml tubes and centrifuged for 10 min at 15,500 rcf. The supernatant was filtered using a 0.22 µm filter to eliminate the cells. The filtered supernatant was frozen at -80°C and freeze-dried in 150 mm Petri dishes for 2 days in a Lyomicron -55oC from Coolvacum Technologies. The freeze-dried material was dissolved in 1 ml of water. To check the inhibition, 10 µl of the dissolved freeze-dried material was placed on a *C. acnes* embedded plate and incubated in anaerobic conditions for 2 to 3 days at 37°C.

### Results

### Microbiome analysis

Microbial face skin samples from 40 individuals were collected and processed as described above. Presence of acne presence and severity were assessed *in situ* by a dermatologist, only grade 1 and 2 (Plewig G. and Kligman A., 1975) acne patients were included. DNA was extracted and sequenced using Illumina MISeq 2x300 Next Generation Sequence system using oligonucleotides designed at the V1-V3 regions of the RNA16S gene as described in Meisel et al 2016. Oligonucleotides sequence were: 27F (forward) AGAGTTTGATCCTGGCTCAG (SEQ ID NO: 1) and 534R (reverse) ATTACCGCGGCTGCTGG (SEQ ID NO: 2).

Sequence data obtained from the Illumina platform were analyzed using informatics to compare the relative presence of bacterial species in acne individuals versus healthy. Spearman's non-parametric correlation was employed to find species more abundant in healthy than in acne individuals representing putative balancers or protectors from acne. A list of the most differently represented species was produced. The top 10 candidates showing a higher difference between healthy and acne patients, which included *Fusobacterium nucleatum,* were tested for *in vitro* inhibition of *C*. *acnes.*3

### Fusobacterium nucleatum inhibits Cutibacterium acnes in vitro

The commercially available strain of *F. nucleatum* ATCC 10953 was grown in agar Nissui medium for 48h at 37°C in anaerobic conditions. Cells were harvested by scraping with 1 ml BM and concentrated by centrifugation (14,100 rcf for 1 min) to a density of 50 optical density (OD) units (measured as absorbance at λ=600 nm in 1 cm cuvettes). 10 µl of concentrated *F. nucleatum* as before were spotted on BMSA plates where *C. acnes* was recently embedded during the plate solidification, as explained above. Plates were incubated in anaerobic conditions by placing them into an anaerobic jar at 37°C for 48 h. *C. acnes* growing inhibition zones were found where *F. nucleatum* was spotted (see Fig.1). Finally, to discard artifacts derived from the bacterial growth, *C. acnes* growth inhibition was also not observed when a distinct bacteria species was used (Fig. 2, control species, which was *C. acnes* on the *C. acnes* culture). Altogether, the results demonstrate that *F. nucleatum* is capable of inhibiting the growth of *C. acnes,* and that this inhibition by *F. nucleatum* is specific.

### F. nucleatum specifically inhibits C. acnes growth and not other species

The ability of *F. nucleatum* to inhibit the growth of other bacterial species further than *C*. *acnes* was tested by using the same approach as before. In Fig. 2 it is shown that *F*. *nucleatum* did not inhibit the growth of either a gram-negative bacteria, such as *E. coli,* or gram-positive such as *Enterococcus faecalis* or *Staphylococcus epidermidis.*

Thus, these results show that *F. nucleatum* provides a very specific inhibition of *C*. acnes, which makes it suitable for treating acne without generating undesired side effects caused by the alteration of the skin microbiota.

### The inhibition by F. nucleatum is more remarkable than the inhibition produced by probiotics used to treat acne

Several well-recognized probiotic bacterial species such as *Bifidobacterium longum, Lactobacillus plantarum, Streptococcus salivarius, Lactobacillus rhamnosus,* and *Lactobacillus reuteri* were included in the assay. As can be appreciated in Fig. 3, none of them were able to inhibit the growth of *C. acnes.*

The ability of a commensal bacterium such as *Prevotella intermedia* to *in vitro* inhibits *C. acnes* has been previously reported. However, the results herein provided pointed to the toxic effect of the hemin group, a component of the *P*. *intermedia* growing medium, on *C. acnes.*

### The inhibitory compound produced by F. nucleatum is secreted to the medium.

*F. nucleatum* was grown in a Nissui plate O/N, collected, and grown in liquid BM for 48h at 37°C, cells were eliminated by centrifugation (15,500 rcf for 10 min). The supernatant was freeze-dried and the correspondent to 50 ml of the supernatant was resuspended in 1 ml of water. 10 µl of the solution were spotted on plates where *C. acnes* was embedded immediately before, identically as above. As can be appreciated in Fig. 4, the inhibitory activity remains in the reconstituted freeze-dried supernatant, suggesting that the inhibitory compound produced by *F. nucleatum* is secreted to the growing medium.

The results here provided clearly show that *F. nucleatum,* or its culture supernatant (i.e., conditioned medium), are capable of inhibiting *C. acnes* growth and, thus, are suitable for treating acne without generating any undesired effect resulting from the inhibition of other bacteria from the skin microbiota.

### Citation List

Fierer, N., Lauber, C.L., Zhou, N., McDonald, D., Costello, E.K., and Knight, R. "Forensic identification using skin bacterial communities", 2010, Proc Natl Acad Sci USA, vol. 107, pp. 6477-6481.

Meisel JS, Hannigan GD, Tyldsley AS, SanMiguel AJ, Hodkinson BP, Zheng Q and Grice EA. "Skin microbiome surveys are strongly influenced by experimental design", 2016, J Invest Dermatol., vol. 136(5), pp. 947-956.

Plewig G, Kligman A., "Acne: morphogenesis and treatment", 1975, New York: Springer-Verlag; pp. 162-3.

## Claims

1. *Fusobacterium nucleatum,* or a lysate thereof, or a culture supernatant thereof, for use in the prevention and/or treatment of a bacterial infection.

2. The *Fusobacterium nucleatum,* or a lysate thereof, or a culture supernatant thereof, for use according to claim 1, wherein the bacterial infection is caused by or associated with *Cutibacterium acnes.*

3. The *Fusobacterium nucleatum,* or a lysate thereof, or a culture supernatant thereof, for use according to any of claims 1-2, wherein the bacterial infection caused by or associated with *Cutibacterium acnes* is selected from the group consisting of acne vulgaris, ocular infection, dental infection, skin infection, endocarditis, bone infection, joint infection, central nervous system infection, sepsis, postoperative infection, device-related infection, and combinations thereof.

4. The *Fusobacterium nucleatum,* or a lysate thereof, or a culture supernatant thereof, for use according to any of claims 1-3, which is for the prevention and/or treatment of acne vulgaris.

5. The *Fusobacterium nucleatum,* or a lysate thereof, or a culture supernatant thereof, for use according to any of claims 1-4, wherein the *Fusobacterium nucleatum* culture supernatant is a cell-free supernatant and/or the *Fusobacterium nucleatum* lysate is a cell-free lysate.

6. The *Fusobacterium nucleatum,* or a lysate thereof, or a culture supernatant thereof, for use according to any of claims 1-5, wherein the lysate is obtainable by a method comprising the steps of:
a) culturing the *Fusobacterium nucleatum* under suitable growth conditions, particularly anaerobic conditions, thereby generating a cell culture; and
b) lysing the cell culture thereby obtaining the lysate.

7. The *Fusobacterium nucleatum,* or a lysate thereof, or a culture supernatant thereof, for use according to any of claims 1-6, wherein the culture supernatant is obtainable by a method comprising the steps of:
a) culturing the *Fusobacterium nucleatum* in a culture medium under suitable growth conditions, particularly anaerobic conditions; and
b) separating the culture medium from the *Fusobacterium nucleatum,* thereby obtaining the culture supernatant.

8. The *Fusobacterium nucleatum,* or a lysate thereof, or a culture supernatant thereof, for use according to any of claims 1-7, wherein *Fusobacterium nucleatum* is selected from the group of subspecies consisting of *Fusobacterium nucleatum subsp. polymorphum, Fusobacterium nucleatum subsp. nucleatum, Fusobacterium nucleatum subsp. vincentii, Fusobacterium nucleatum subsp. fusiforme, Fusobacterium nucleatum subsp. animalis,* and combinations thereof.

9. The *Fusobacterium nucleatum,* or a lysate thereof, or a culture supernatant thereof, for use according to claim 8, wherein *Fusobacterium nucleatum* is *Fusobacterium nucleatum subsp. polymorphum.*

10. The *Fusobacterium nucleatum,* or a lysate thereof, or a culture supernatant thereof, for use according to claim 9, wherein the *Fusobacterium nucleatum subsp. polymorphum* is the strain ATCC10953 or a mutant thereof which retains or enhances the capacity of the parent strain to inhibit *C. acnes* growth.

11. A pharmaceutical composition comprising a therapeutically effective amount of the *Fusobacterium nucleatum,* or a lysate thereof, or a supernatant thereof, together with at least one pharmaceutically acceptable excipient and/or carrier.

12. The pharmaceutical composition according to claim 11, which is for use in the prevention and/or treatment of a bacterial infection, particularly a bacterial infection caused by or associated with *Cutibacterium acnes.*

13. A cosmetic composition comprising a cosmetically effective amount of *Fusobacterium nucleatum,* or a lysate thereof, or a supernatant thereof, together with at least one cosmetically acceptable excipient and/or carrier.

14. A non-therapeutic use of *Fusobacterium nucleatum,* or a lysate thereof, or a supernatant thereof, as an antibacterial agent; particularly, for inhibiting *Cutibacterium acnes* growth.

15. The pharmaceutical composition according to claim 11, the pharmaceutical composition for use according to claim 12, the cosmetic composition according to claim 13, or the non-therapeutic use according to claim 14, wherein *Fusobacterium nucleatum* is *Fusobacterium nucleatum subsp. polymorphum.*
